# EUROPEAN PATENT SPECIFICATION

(11) **EP 3 841 136 B1**
(45) Date of publication and mention of the grant of the patent: **19.06.2024**
(21) Application number: 19749764.7
(22) Date of filing: 12.08.2019
(51) Int. Cl.: C08G 18/48, C08G 18/76, C08G 18/22, C08G 101/00, C08G 18/18, C08G 18/20, C08G 18/09

(54) **CATALYST FOR PIR/PUR FOAM PRODUCTION**
KATALYSATOR ZUR PIR-/PUR-SCHAUMHERSTELLUNG
CATALYSEUR POUR LA PRODUCTION DE MOUSSE PIR/PUR

(30) Priority: 21.08.2018 EP 18189859; 06.02.2019 EP 19155718; 10.05.2019 EP 19173695
(43) Date of publication of application: 30.06.2021
(73) Proprietor: Huntsman International LLC, The Woodlands, TX 77380 (US)
(72) Inventor: BUONO, Pietro, 1200 Woluwe-Saint-Lambert (BE); DRIES, Geert Lodewijk, 3520 Zonhoven (BE); HUMBERT, Heiko Heinrich, 21075 Hamburg (DE); VANDERSTRAETEN, Petra Emma, 3000 Leuven (BE)
(74) Representative: Roberts, Philippa Grace
(86) International application number: PCT/EP2019/071592
(87) International publication number: WO 2020/038755

(56) References cited:
- WO-A1-2016/051193
- WO-A1-2017/160362
- US-A1- 2001 008 948
- US-A1- 2004 238 786
- US-A1- 2007 161 524
- US-A1- 2009 005 355
- US-A1- 2011 033 354
- NICOLA OTTO ET AL: "Screening of ligands for the Ullmann synthesis of electron-rich diaryl ethers", BEILSTEIN JOURNAL OF ORGANIC CHEMISTRY, vol. 8, 17 July 2012 (2012-07-17), pages 1105-1111, XP055557899, DOI: 10.3762/bjoc.8.122

## Description

### TECHNICAL FIELD

The present disclosure is directed to a process for the synthesis of a catalyst for PIR/PUR foam production, uses of such catalyst and a process for PIR/PUR foam production.

### BACKGROUND ART

Polyisocyanurate/polyurethane (PIR/PUR) foams are generally formulated by the reaction of a polyester polyol and 4,4'-methylenedi(phenylisocyanate) (MDI) in the presence of a suitable catalyst as well as additional additives such as blowing agents and surfactants. The starting materials are similar to those used in PUR foam production except that the proportion of MDI is higher and a polyester polyol is used instead of a polyether polyol. The resulting material presents a significantly different chemical structure with the isocyanate groups on the MDI trimerizing to form isocyanurate groups which the polyols link together, resulting in a complex polymeric structure as outlined below.

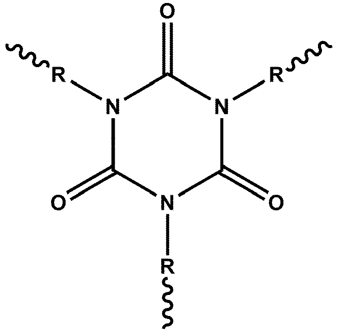

PIR/PUR foams have been acknowledged to possess excellent thermal stability and fire resistance as well as a very good chemical stability to widely used organic solvents, acids, alkali, moisture and UV light.

Potassium acetate, a low molecular weight carboxylate salt, as well as potassium octoate have been widely used as catalysts for the production of rigid PIR/PUR foams. However, the use of commercially available alkali metal carboxylate salt catalysts often has the drawback of a limited control of the rise speed profile of the foam, leading to undesired processing problems like foam overpacking, foam backflowing and overheating due to the strong exothermic reaction.

A much better controlled rise profile has been obtained with sterically hindered alkali metal carboxylate salts (WO 2016/201675 A1) and hexahydrotriazine carboxylates (US 4,310,633, EP 1777242 A1, WO 2017/160362 A1). However, a controlled rise profile might result in an undesirably slow process, which will inevitably affect the productivity.

The development of polyisocyanurate catalysts based on alkylammonium carboxylate salts (US 4,186,255) shows different foam processability, providing a smoother rate of rise profile. However, alkylammonium carboxylates can be unstable above 100 °C, causing the release of volatile amines imparting undesirable odors to the final foam product.

A number of patents and patent applications (US 3,896,052, US 3,896,991, US 3,903,018, US 4,011,180, US 4,101,465, US 4,623,673, US 5,844,012, US 2004/0259967 A1 and EP 1777242 A1) describe alkali metal salts of N,N-(2-hydroxyphenyl)methylglycine, in most cases with a further substitution on the phenyl ring and on the nitrogen atom, such as alkali metal salts of N-(2-hydroxy-5-nonylphenyl)methyl-N-methylglycine, as catalysts, alone or in a co-catalyst system, for PIR/PUR foam production.

Therefore, there is a need for the development of a catalyst that can offer a smooth rise profile for the manufacture of rigid PIR/PUR foams and at the same time provide a good thermal stability at the usual processing temperatures, thus avoiding the evolution of malodorous low molecular weight amines.

### DISCLOSURE

Unless otherwise defined herein, technical terms used in connection with the present disclosure shall have the meanings that are commonly understood by those having ordinary skill in the art. Further, unless otherwise required by context, singular terms shall include pluralities and plural terms shall include the singular.

All patents, published patent applications, and non-patent publications mentioned in the specification are indicative of the level of skill of those skilled in the art to which the present disclosure pertains.

As utilized in accordance with the present disclosure, the following terms, unless otherwise indicated, shall be understood to have the following meanings.

The use of the word "a" or "an", when used in conjunction with the term "comprising", "including", "having", or "containing" (or variations of such terms) may mean "one", but it is also consistent with the meaning of "one or more", "at least one", and "one or more than one".

The use of the term "or" is used to mean "and/or" unless clearly indicated to refer solely to alternatives and only if the alternatives are mutually exclusive.

Throughout this disclosure, the term "about" is used to indicate that a value includes the inherent variation of error for the quantifying device, mechanism, or method, or the inherent variation that exists among the subject(s) to be measured. For example, but not by way of limitation, when the term "about" is used, the designated value to which it refers may vary by plus or minus ten percent, or nine percent, or eight percent, or seven percent, or six percent, or five percent, or four percent, or three percent, or two percent, or one percent, or one or more fractions therebetween.

The use of "at least one" will be understood to include one as well as any quantity more than one, including but not limited to, 1, 2, 3, 4, 5, 10, 15, 20, 30, 40, 50, 100, etc. The term "at least one" may extend up to 100 or 1000 or more depending on the term to which it refers. In addition, the quantities of 100/1000 are not to be considered as limiting since lower or higher limits may also produce satisfactory results.

As used herein, the words "comprising" (and any form of "comprising", such as "comprise" and "comprises"), "having" (and any form of "having", such as "have" and "has"), "including" (and any form of "including", such as "includes" and "include") or "containing" (and any form of "containing", such as "contains" and "contain") are inclusive or open-ended and do not exclude additional, unrecited elements or method steps.

The phrases "and mixtures thereof", "or combinations thereof" and "and combinations thereof" as used herein refers to all permutations and combinations of the listed items preceding the term. For example, "A, B, C, or combinations thereof" is intended to include at least one of: A, B, C, AB, AC, BC, or ABC and, if order is important in a particular context, also BA, CA, CB, CBA, BCA, ACB, BAC, or CAB. Continuing with this example, expressly included are combinations that contain repeats of one or more items or terms such as BB, AAA, CC, AABB, AACC, ABCCCC, CBBAAA, CABBB, and so forth. The skilled artisan will understand that typically there is no limit on the number of items or terms in any combination, unless otherwise apparent from the context. In the same light, the terms "or combinations thereof" and "and combinations thereof" when used with the phrases "selected from" or "selected from the group consisting of" refers to all permutations and combinations of the listed items preceding the phrase.

The phrases "in one embodiment", "in an embodiment", "according to one embodiment", and the like generally mean the particular feature, structure or characteristic following the phrase is included in at least one embodiment of the present disclosure, and may be included in more than one embodiment of the present disclosure. Importantly, such phrases are non-limiting and do not necessarily refer to the same embodiment but, of course, can refer to one or more preceding and/or succeeding embodiments. For example, in the appended claims, any of the claimed embodiments can be used in any combination.

The terms "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the present disclosure.

According to the invention, the term PIR/PUR foam refers to a foam material comprising polyurethane (PUR) and/or polyisocyanurate (PIR) structures, said PIR/PUR foam may be a rigid, a semi-rigid (semi-flexible) or a flexible foam.

The present disclosure is related to a process for the synthesis of a compound according to general formula (I): 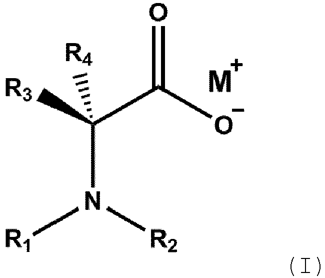 wherein R₁ and R₂ are independently selected from C₁-C₁₈ straight-chain or branched alkyl groups, unsubstituted or substituted with one or more hydroxyl, amino or aminoalkyl groups, or R₁ and R₂, taken together, form a 5- or 6-membered ring or 7-membered bicyclic structure, one of the members of the ring or bicyclic structure being X, wherein X is selected from CH₂, O, S, NCH₃ or NCH₂COOM, wherein R₃ and R₄ are independently selected from hydrogen or C₁-C₄ straight-chain or branched alkyl groups , and M is an alkali metal ion or a quaternary ammonium ion, the process comprising the steps according to claim 1. In case of R₁ and R₂ taken together to form a ring or bicyclic structure, the compound therefore has the formula (II) as below: 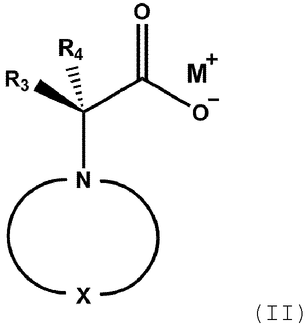 wherein, in the case of a bicyclic structure, the ring comprises an additional bridge.

In a preferred embodiment of the present disclosure, R₃ and R₄ are both hydrogen.

If R₁ and/or R₂ are substituted alkyl groups, they preferably comprise at least one terminal hydroxyl, amino or aminoalkyl group.

In another embodiment of the present disclosure, R₁ and R₂ are identical.

In one specifically preferred embodiment, R₁ and R₂ are both 2-hydroxyethyl.

If R₁ and R₂ are taken together to form a ring, such ring is preferably selected from a 5- or 6-membered ring or 7-membered bicyclic structure with X being CH₂, a 6-membered ring with X being O or a 6-membered ring with X being NCH₂COOM. However, these cyclic compounds are less preferred because of lower yields.

In a preferred embodiment of the present disclosure, M is an alkali metal ion, most preferably a potassium ion. In other embodiments the alkali metal ion M is a lithium ion, a sodium ion or combinations thereof with the potassium ion.

In a preferred embodiment of the present disclosure, the compound of general formula (I) is selected from potassium *N,N-*bis(2-hydroxyethyl)glycinate, potassium N-(2-hydroxyethyl)-N-methylglycinate, potassium N,N-bis(3-(dimethyl-amino) propyl) glycinate, potassium N,N-dibutylglycinate or combinations thereof.

In the presently most preferred embodiment of the disclosure, the compound of general formula (I) is potassium N,N-bis(2-hydroxyethyl)glycinate.

Although less preferred, potassium 2-(pyrrolidin-1-yl)acetate, potassium 2-morpholinoacetate, potassium 2,2'-(piperazine-1,4-diyl)diacetate, potassium 2-(2-azabicyclo[2.2.1]heptane-2-yl)acetate or combinations thereof may also be used.

The process according to the present invention comprises the steps:
(i) reacting a secondary amine having the general formula R₁-NH-R₂ and a dicarbonyl compound of the general formula R₃-CO-CO-R₄ under appropriate reaction conditions to form a N,N-disubstituted glycine compound, and
(ii) neutralizing the reaction product of step (i) by adding a solution of an alkali or tetraalkylammonium hydroxide having the general formula MOH to form a compound according to general formula (I), wherein R₁, R₂, R₃, R₄ and M are defined as hereinabove.

Preferably, R₃ and R₄ are both hydrogen, i.e. the dicarbonyl compound is glyoxal.

In embodiments of the present disclosure, the dicarbonyl compound can be also selected from 2-oxopropanal, biacetyl, pentane-2,3-dione, hexane-3,4-dione, heptane-2,3-dione or combinations thereof. These compounds are exemplified in pictures below.

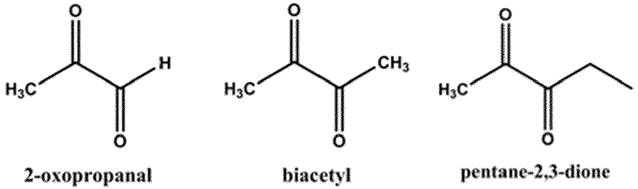

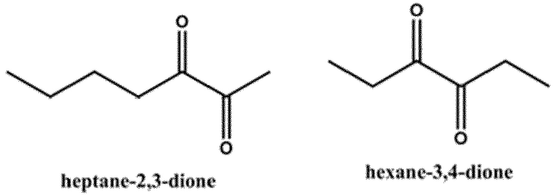

Furthermore, the present disclosure is related to the use of a compound of general formula (I) as defined hereinabove as a catalyst or co-catalyst in a rigid, semi-rigid or flexible PIR/PUR foam production.

In one specifically preferred embodiment of such use, the compound according to general formula (I) is potassium N,N-bis(2-hydroxyethyl)glycinate

Finally, the present disclosure is related to a process for producing a rigid, semi-rigid or flexible PIR/PUR foam comprising reacting a polyol or polyol blend comprising polyester and/or polyether polyols with polyisocyanates such as 4,4'-methylenedi(phenylisocyanate) (MDI) in the presence of a catalyst according to the present disclosure and optionally further additives under appropriate reaction conditions.

### DETAILED DESCRIPTION

A whole range of *N,N*-disubstituted glycinate salts are covered by the above definition and can be used as catalysts for PIR/PUR foam production according to the present disclosure.

Such glycinate salts may be asymmetrically substituted compounds such as, for example, R₁ being methyl and R₂ being 2-hydroxyethyl.

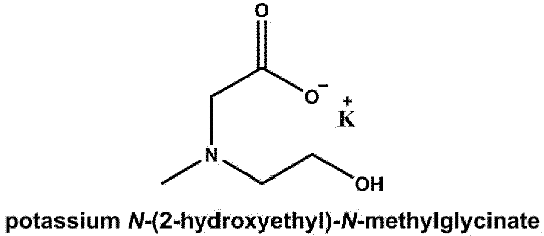

In a presently preferred embodiment, the substituents on the nitrogen atom of the glycine moiety are identical, for example, 2-hydroxyethyl, butyl or *N,N*-dimethylpropylamine. Some embodiments of the present invention are shown below.

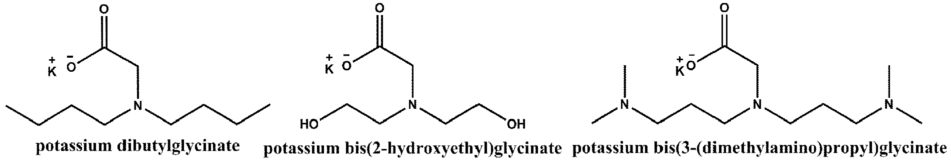

Examples for a cyclic substitution can be as follows

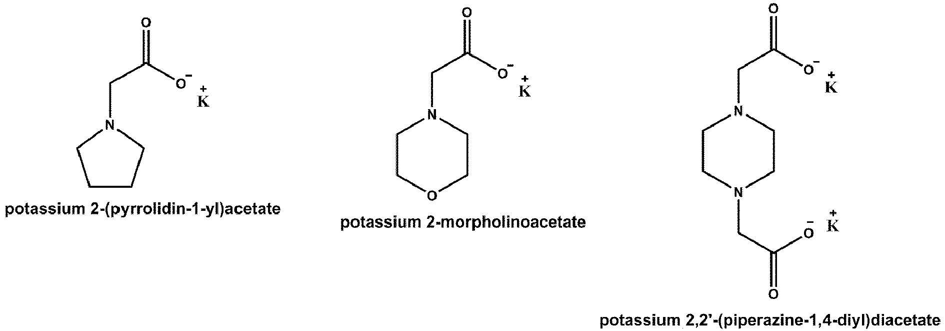

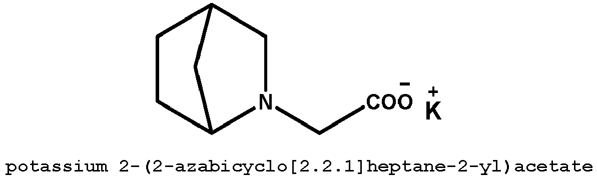

However, it has to be emphasized the all compounds falling under the above definition are appropriate for use as catalysts for PIR/PUR foam production.

R₁ and R₂ in structure (I) may in some embodiments be independently selected from a C₄-C₁₈ straight-chain or branched unsubstituted alkyl group, in other embodiments from a C₅-C₁₅ straight-chain or branched unsubstituted alkyl group, in even other embodiments, R₁ and R₂ may be independently selected from a C₉-C₁₅ straight-chain or branched unsubstituted alkyl group.

In still other embodiments, R₁ and R₂ in structure (I) may be independently selected from a C₄-C₁₈ straight-chain or branched alkyl group substituted with one or more hydroxyl, amino or aminoalkyl groups, in other embodiments from a C₅-C₁₅ straight-chain or branched alkyl group substituted with one or more hydroxyl, amino or aminoalkyl groups, in even other embodiments, R₁ and R₂ may be independently selected from a C₉-C₁₅ straight-chain or branched alkyl group substituted with one or more hydroxyl, amino or aminoalkyl groups.

In some embodiments, R₁ and R₂ in structure (I) are taken together to form a ring or bicyclic structure comprising a azetidinyl, a pyrazolidinyl, a imidazolidinyl, a piperazinyl, thiomorpholinyl, thiomorpholinyl dioxide, decahydroisoquinolinyl, decahydroquinolinyl, 2-azaadamantanyl or azocanyl or substructure.

In a preferred embodiment, a catalyst for PIR/PUR foam production comprises a compound having the general formula (I), wherein R₁ and R₂ are independently selected from a C₄-C₁₈ straight-chain or branched unsubstituted alkyl group, or a C₁-C₁₈ straight-chain or branched alkyl group substituted with one or more hydroxyl, amino or aminoalkyl groups, or R₁ and R₂, taken together, form a 5- or 6-membered ring or 7-membered bicyclic structure, one of the members of the ring or bicyclic structure being X, wherein X is selected from CH₂, O, S, NCH₃ or NCH₂COOM, wherein R₃ and R₄ are independently selected from hydrogen or a C₁-C₄ straight-chain or branched alkyl group and wherein M is an alkali metal ion or a quaternary ammonium ion, with the exception of compounds according to structure (I) comprising a pyrrolidinyl, a piperidinyl or a morpholinyl structure.

In another embodiment, a catalyst for PIR/PUR foam production comprises a compound having the general formula (I), wherein R₁ and R₂ are independently selected from a C₄ straight-chain or branched unsubstituted alkyl group, or a C₁-C₄ straight-chain or branched alkyl group substituted with one or more hydroxyl, amino or aminoalkyl groups, or R₁ and R₂, taken together, form a 5- or 6-membered ring or 7-membered bicyclic structure, one of the members of the ring or bicyclic structure being X, wherein X is selected from CH₂, O, S, NCH₃ or NCH₂COOM, wherein R₃ and R₄ are independently selected from hydrogen or a C₁-C₄ straight-chain or branched alkyl group and wherein M is an alkali metal ion or a quaternary ammonium ion, with the exception of compounds according to structure (I) comprising a pyrrolidinyl, a piperidinyl or a morpholinyl structure

In some embodiments, R₁ and R₂ in structure (I), taken together, form a 5- or 6-membered ring or 7-membered bicyclic structure, one of the members of the ring or bicyclic structure being CH₂, in some embodiments O, in other embodiments S, in even other embodiments NCH₃ or in some embodiments NCH₂COOM, with the exception of compounds according to structure (I) comprising a pyrrolidinyl, a piperidinyl or a morpholinyl structure.

The *N,N-*disubstituted glycinate salts used as catalysts in the process of the present disclosure can, in most cases, be prepared by the following two-step synthesis:
1) The first step is a reaction between a secondary amine of the general formula R₁-NH-R₂ and a dicarbonyl compound of the general formula R₃-CO-CO-R₄ at a temperature in the range from 50 to 100°C for 5 to 24 hours. 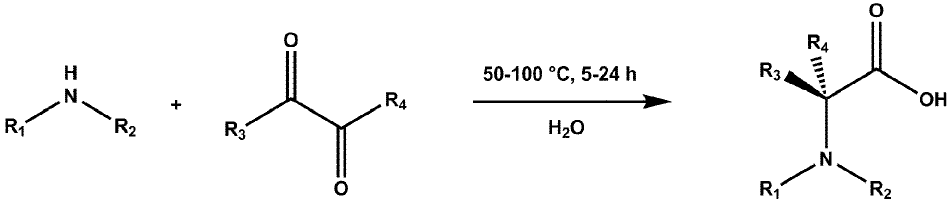
2) The second step is the neutralization of the acid obtained in the first reaction step, by the addition of a solution of alkali hydroxide (e.g. sodium hydroxide (NaOH), potassium hydroxide (KOH)) or tetralkylammonium hydroxide. The resulting *N,N-*disubstituted glycinate salt may be solubilized in diethylene glycol (DEG), dipropylene glycol (DPG), propylene glycol (PG), ethylene glycol (EG), 1,4-butandiol or other suitable polyether polyol to obtain a solution of the N,N-disubstituted glycinate salt in the respective organic solvent for use as a catalyst in the process of production of PIR/PUR foams of the present disclosure.

In some embodiments, the amine of the general formula R₁-NH-R₂, which is reacted with the dicarbonyl compound of the general formula R₃-CO-CO-R₄, is *N,N'*-bis (2-hydroxyethyl)ethylenediamine, a poly(ethyleneimide), a hydroxyethyl-endcapped poly(ethyleneimide) or a JEFFAMINE^{®} Secondary Amine of the SD Series or ST Series by the Huntsman Corporation.

As already mentioned in the introductory part of the present disclosure, PIR/PUR foams are generally formulated by the reaction of a polyester polyol or polyester polyol blend and 4,4'-methylenedi(phenylisocyanate)(MDI) in the presence of a suitable catalyst as well as additional additives such as blowing agents and surfactants.

The term "polyester polyol" as used herein includes any minor amounts of unreacted polyol remaining after the preparation of the polyester polyol and/or unesterified polyol (for example, glycol) added after the preparation of the polyester polyol. Polyester polyols can be produced, for example, from organic dicarboxylic acids with 2 to 12 carbons, including aliphatic dicarboxylic acids with 4 to 6 carbons, and divalent and multivalent alcohols, including diols, with 2 to 12 carbons, such as, 2 to 6 carbons. Examples of dicarboxylic acids include succinic acid, glutaric acid, adipic acid, suberic acid, azelaic acid, sebacic acid, decanedicarboxylic acid, maleic acid, fumaric acid, phthalic acid, isophthalic acid, and terephthalic acid. The dicarboxylic acids can be used individually or in mixtures. Instead of the free dicarboxylic acids, the corresponding dicarboxylic acid derivatives may also be used, such as dicarboxylic acid mono- or di-esters of alcohols with 1 to 4 carbons, or dicarboxylic acid anhydrides. Dicarboxylic acid mixtures of succinic acid, glutaric acid and adipic acid in quantity ratios of 20-35:35-50:20-32 parts by weight may also be used. Examples of divalent and multivalent alcohols include ethanediol, diethylene glycol, 1,2- and 1,3-propanediol, dipropylene glycol, 1,4-butanediol, 1,5-pentanediol, 1,6-hexanediol, 1,10-decanediol, glycerine and trimethylolpropanes, tripropylene glycol, tetraethylene glycol, tetrapropylene glycol, tetramethylene glycol, 1,4-cyclohexanedimethanol, ethanediol, diethylene glycol, 1,4-butanediol, 1,5-pentanediol, 1,6-hexanediol, or mixtures of at least two of these diols, especially mixtures of 1,4-butanediol, 1,5-pentanediol, and 1,6-hexanediol. Furthermore, polyester polyols of lactones, for example, ε-caprolactone or hydroxycarboxylic acids, for example, ω-hydroxycaproic acid, may also be used.

The amount of polyol to be used relative to the isocyanate normally should be such that the isocyanate groups are present in at least an equivalent amount, and preferably, in slight excess, compared to the free hydroxyl groups. In some embodiments, the components will be proportioned so as to provide from about 0.9 to about 1.5 mole equivalents of isocyanate groups per mole equivalent of hydroxyl groups. However, for certain foams the mole equivalents of isocyanate to hydroxyl groups can be as low as 0.4.

Also included in the foam composition may be a blowing agent, which may be selected based in part upon the desired density of the final foam. In certain non-limiting embodiments hydrocarbon blowing agents may be selected. For example, hydrocarbon or fluorine-containing hydrohalocarbon blowing agents may be used, and in some instances may serve to reduce, or further reduce, viscosity, and thereby enhance processability. Among these are, for example, butane, isobutane, 2,3-dimethylbutane, n- and i-pentane isomers, hexane isomers, heptane isomers, cycloalkanes including cyclopentane, cyclohexane, cycloheptane, and combinations thereof, HFC-245fa (1,1,1,3,3-pentafluoropropane), HFC-365mfc (1,1,1,3,3-penta-fluorobutane), HFC-227ea (1,1,1,2,3,3,3-heptafluoropropane), HFC-134a (1,1,1,2-tetrafluoroethane), combinations of two or more of the above, and the like. In one particular embodiment, the blowing agent is an unsaturated halogenated hydroolefin such as a hydrofluoroolefin (HFO), hydrochlorofluoroolefin (HCFO), or mixtures thereof. Preferred hydrofluoroolefin (HFO) blowing agents contain 3, 4, 5, or 6 carbons, and include but are not limited to pentafluoropropenes, such as 1,2,3,3,3-pentafluoropropene (HFO-1225ye); tetrafluoropropenes, such as 1,3,3,3-tetrafluoropropene (HFO-1234ze, E and Z isomers), 2,3,3,3-tetrafluoropropene (HFO-1234yf), and 1,2,3,3-tetrafluoropropene (HFO-1234ye); trifluoropropenes, such as 3,3,3-trifluoropropene (HFO-1243zf); tetrafluorobutenes, such as (HFO- 1345); pentafluorobutene isomers, such as (HFO-1354); hexafluorobutene isomers, such as (HFO-1336); heptafluorobutene isomers, such as (HFO-1327); heptafluoropentene isomers, such as (HFO-1447); octafluoropentene isomers, such as (HFO-1438); nonafluoropentene isomers, such as (HFO-1429); and hydrochlorofluoroolefins, such as 1-chloro-3,3,3-trifluoropropene (HCFO-1233zd) (E and Z isomers), 2-chloro-3,3,3-trifluoropropene (HCFO-1233xf), HCFO-1223, 1,2-dichloro-1,2-difluoroethene (E and Z isomers), 3,3-dichloro-3-fluoropropene, 2-chloro-1,1,1,4,4,4-hexafluorobutene-2 (E and Z isomers), and 2-chloro-1,1,1,3,4,4,4-heptafluorobutene-2 (E and Z isomers). These hydrocarbons and/or non-fluorine-containing hydrohalocarbons may be used in an amount of about 0.5 parts by weight to about 30 parts by weight, or about 1 part by weight to about 10 parts by weight, based on 100 parts by weight of polyol(s).

Another blowing agent that may be used is formic acid or another carboxylic acid. The formic acid or other carboxylic acid may be used in an amount of from about 0.5 parts by weight to about 8 parts by weight, based on 100 parts by weight of polyol(s). It is also contemplated that other aliphatic mono- and polycarboxylic acids may be employed, such as those disclosed in US 5,143,945, and including isobutyric acid, ethylbutyric acid, ethylhexanoic acid, and combinations thereof.

In addition to, or in lieu of, the blowing agents above, water may also be optionally selected as a blowing agent. The water is, in some non-limiting embodiments, present in an amount of from about 0.1 parts by weight to about 40 parts by weight, or from about 0.15 parts by weight to about 20 parts by weight, or from about 0.25 parts by weight to about 5 parts by weight, or from about 0.5 parts by weight to about 3 parts by weight, based on 100 parts by weight of polyol(s). In some embodiments, when preparing a PIR/PUR foam, in order to facilitate and give desirable processing characteristics, the amount of water used may not exceed 3 parts by weight water, or not more than 2.5 parts by weight of water, or not more than 1 part by weight of water, based on 100 parts by weight of polyol(s).

In addition to the catalyst of the present disclosure, other catalysts may optionally be included in the foam composition.

For example, an amine catalyst may be included, including any organic compound which contains at least one tertiary nitrogen atom and is capable of catalyzing the hydroxyl/isocyanate reaction between the isocyanate and polyol. Typical classes of amines include the N-alkylmorpholines, N-alkyl-alkanolamines, N,N-dialkylcyclohexylamines, and alkylamines where the alkyl groups are methyl, ethyl, propyl, butyl and isomeric forms thereof, and heterocyclic amines. Typical but non-limiting thereof are triethylenediamine, tetramethylethylenediamine, bis(2-dimethylaminoethyl)ether, triethylamine, tripropylamine, tributylamine, triamylamine, pyridine, quinoline, dimethylpiperazine, piperazine, N,N-dimethylcyclohexylamine, N-ethyl-morpholine, 2-methylpropanediamine, methyltriethyl-enediamine, 2,4,6-tridimethylamino-methyl)phenol, N,N',N"-tris(dimethylaminopropyl)sym-hexahydrotriazine, and mixtures thereof. The tertiary amines from which selection may be made may include bis(2-dimethylamino-ethyl)ether, dimethylcyclohexylamine, N,N-dimethyl-ethanolamine, triethylenediamine, triethylamine, 2,4,6-tri(dimethylaminomethyl)phenol, N,N',N-ethylmorpholine, and mixtures thereof.

Non-amine catalyst may also be included in foam compositions of the present disclosure. Typical of such catalysts are organometallic compounds of bismuth, lead, tin, titanium, iron, antimony, uranium, cadmium, cobalt, thorium, aluminum, mercury, zinc, nickel, cerium, molybdenum, vanadium, copper, manganese, zirconium, and combinations thereof. Included for illustrative purposes only are bismuth nitrate, lead 2-ethylhexoate, lead benzoate, lead naphthenate, ferric chloride, antimony trichloride, antimony glycolate, combinations thereof, and the like. Embodiments include the stannous salts of carboxylic acids, such as stannous acetate, stannous octoate, stannous 2-ethylhexoate, 1-methylimidazole, and stannous laurate, as well as the dialkyl tin salts of carboxylic acids, such as dibutyl tin diacetate, dibutyl tin dilaurate, dibutyl tin dimaleate, dioctyl tin diacetate, combinations thereof and the like.

The amount of catalyst (and optional catalyst(s)) added to the foam composition is a catalytically sufficient amount. In one embodiment, the amount of catalyst substantially free of nonylphenol (and optional catalyst(s)) that is included in the foam composition is from about 0.5 parts by weight to about 15 parts by weight, in another embodiment from about 0.75 part by weight to about 12 parts by weight, and in still another embodiment from about 1 part by weight to about 10 parts by weight, based on 100 parts by weight of polyol(s).

If desired, the foam composition can also comprise additives. Examples include: cell stabilizers, such as organopolysiloxane surfactants; flame retardants such as halogenated organophosphorous compounds; chain extenders such as ethylene glycol and butane diol; crosslinkers such as glycerol, trimethylolpropane, pentaerythritol, sucrose, sorbitol, or mixtures thereof; fillers and pigments, such as calcium carbonate, titanium dioxide, iron oxide, chromium oxide, azo/diazo dyes, phthalocyanines, dioxazines, carbon black barium sulfate, graphite, microspheres, alumina trihydrate, wollastonite, prepared glass fibers (dropped or continuous), and polyester fibers and other polymeric fibers, as well as various combinations thereof; odor masks, biocides, antioxidants, UV stabilizers such as hydroxybenzotriazoles, zinc dibutyl thiocarbamate, 2,6-ditertiarybutyl catechol, hydroxybenzophenones, hindered amines and phosphites; antistatic agents; viscosity modifiers and combinations thereof.

Each additive, except fillers and pigments, may constitute from 0.01 parts by weight to about 10 parts by weight, based on 100 parts by weight of polyol(s). Fillers and pigments may be used in quantities as high as up to about 50 parts by weight, based on 100 parts by weight of polyol(s).

The preparation of PIR/PUR foams may follow any of the methods well known in the art for preparing such type of foam, for example, as described in Saunders and Frisch, "Volumes I and II Polyurethanes Chemistry and Technology" (1962, John Wiley and Sons, New York, N.Y.), or Gum et al., "Reaction Polymers" (1992, Oxford University Press, New York, N.Y.), or Klempner and Sendijarevic, "Polymeric Foams and Foam Technology" (2004, Hanser Gardner Publications, Cincinnati, Ohio). In general, the various components are brought together and mixed using manual or mechanical mixing means to form the foam. Generally, the polyol and catalyst are preblended to form a single component which is fed as one stream to a conventional mixing head and admixed with the isocyanate which is fed as a separate stream to the mixing head. The blowing agent can be fed as a separate stream to the mixing head or blended with one or other, or both, of the other components prior to feeding the latter to the mixing head.

The foams subsequently produced can be rigid, flexible, or semi-rigid, and can have a closed cell structure, an open cell structure or a mixture of open and closed cells and may vary in density of from about 0.5 pounds per cubic foot to about 60 pounds per cubic foot, such as from about 1.0 to 20.0 pounds per cubic foot, and in other embodiments from about 1.5 to 6.0 pounds per cubic foot. The foams are suitable for use in a wide range of applications including appliance insulation (e.g., insulating refrigerators or water heaters), structural insulation (e.g. spray foams or lamination foams for commercial or residential insulation), cushioning, flotation, packaging, adhesives, void filling, crafts and decorative, and shock absorption.

The PIR/PUR foam prepared according to the process of this disclosure is a rigid, closed-cell foam. Such foam is typically prepared by intimately mixing the reaction components, for example, a B-side containing a polyol/blowing agent/catalyst according to the present disclosure and an A-side containing an isocyanate (i.e., two streams); or a B-side containing a polyol/blowing agent, a C-side containing the catalyst according to the present disclosure, and an A-side containing a polyisocyanate component (at least three streams, wherein the polyol/blowing agent and catalyst according to the present disclosure mix just prior to contact thereof with the polyisocyanate component) at room temperature or at a slightly elevated temperature for a short period of time. Mixing of streams may be carried out either in a spray apparatus, a mixhead with or without a static mixer, or a vessel. The mixture is then sprayed or otherwise deposited onto a substrate. This substrate may be, for example, a rigid or flexible facing sheet made of foil or another material, including another layer of similar or dissimilar polyurethane or polyisocyanurate which is being conveyed, continuously or discontinuously, along a production line, or directly onto a conveyor belt.

The result may be a rigid foam in the form of slabstock, a molding, a filled cavity, including but not limited to a pipe or insulated wall or hull structure, a sprayed foam, a frothed foam, or a continuously- or discontinuously-manufactured laminate product, including but not limited to a laminate or laminated product formed with other materials, such as hardboard, plasterboard, plastics, paper, metal, or a combination thereof.

The advantages of the catalysts and PIR/PUR foams disclosed are further illustrated by the following examples. However, the following non-limiting examples are not intended to be all-inclusive and are not intended to limit the scope of the embodiments described herein.

### SYNTHESIS EXAMPLES

**Diethanolamine:** obtained from The Dow Chemical Company.
**Glyoxal solution 40% in water:** obtained from BASF.
**Bis(2-hydroxyethyl)glycine:** obtained from TCI Europe.
**2-(Methylamino)ethanol:** obtained from TCI Europe.
**Dibutylamine:** obtained from TCI Europe.
**JEFFCAT^{®} Z-130 catalyst:** N¹-(3-(dimethylamino)propyl)-N³,N³-dimethylpropane-1,3-diamine obtained from Huntsman Corporation.
**Glycine:** obtained from TCI Europe.
**Cyclopentadiene dimer:** obtained from The Dow Chemical Company
**Formaline solution 37% in water:** obtained from VWR International.
**Palladium on carbon (5% Pd):** obtained from Johnson Mattey.

### EXAMPLE 1:

As one preferred example for the catalyst of the present disclosure, the potassium salt of bis(2-hydroxyethyl) glycinate (BHEGLY)of the following formula: 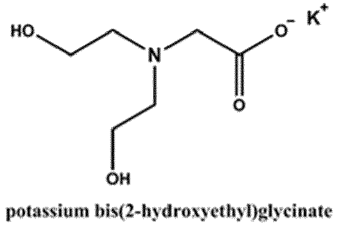 has been synthesized as follows:
1)106 grams (g.) (1 mole) of diethanolamine were diluted in 200 milliliters (mL) of distilled water and charged in a 1 liter (L) three necked flask equipped with a thermometer, condenser and a magnetic stirring bar. Then, 145.1 g. (1 mole) of glyoxal 40% wt solution in water was added at room temperature through a dropping funnel. The mixture was then heated up at about 70°C and stirred for about 16 hours as shown below (Yield 94%). 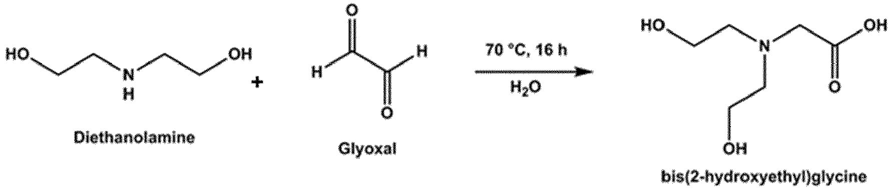
2) The second step was the neutralization of the acid obtained in the first reaction step by the addition of 112.2 g of potassium hydroxide (1 mole KOH), a 50% wt solution of potassium hydroxide (KOH) in water as the alkali hydroxide.

The resulting glycinate salt is solubilized in diethylene glycol (DEG) to obtain a solution of glycinate salt in DEG for use as a catalyst in the process of production of PIR/PUR foams of the present disclosure is exemplified in the following Application Example.

### EXAMPLE 2:

The potassium diethanol glycinate solution in DEG illustrated in Example 1 has been prepared also from commercially available bis(2-hydroxyethyl)glycine: 163.2 g (1 mole) of diethanolglycine were charged in a 1 L three necked flask equipped with a thermometer, condenser and a magnetic stirring bar. Then 112.2 g (1 mole) of potassium hydroxide (KOH), a 50% wt solution of potassium hydroxide (KOH) in water, were added and the mixture stirred for 1 hour at room temperature (100% yield).

The resulting glycinate salt is solubilized in diethylene glycol (DEG) to obtain a solution of glycinate salt in DEG for use as a catalyst in the process of production of PIR/PUR foams.

### EXAMPLE 3:

The potassium salt of bis(3-(dimethyl-amino)propyl)glycine of the following formula has been prepared as follows: 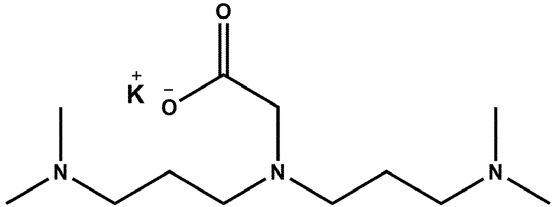
1) 95 g (0.5 moles) of JEFFCAT Z-130^{®} catalyst (N¹-(3-(dimethylamino)propyl)-N³,N³-dimethylpropane-1,3-diamine) were diluted in 150 mL of distilled water and charged in a 1 L three necked flask equipped with a thermometer, condenser and a magnetic stirring bar. Then, 72.6 g (0.5 mole) of glyoxal 40% wt solution in water was added at room temperature through a dropping funnel. The mixture was then heated up at a temperature of about 70°C and stirred for about 16 hours (Yield 92%).
2) The second step was the neutralization of the acid obtained in the first reaction step by the addition of 56.1 g of potassium hydroxide (KOH), a 50% wt solution of potassium hydroxide (KOH) in water as the alkali hydroxide.

The resulting glycinate salt is solubilized in diethylene glycol (DEG) to obtain a solution of glycinate salt in DEG for use as a catalyst in the process of production of PIR/PUR foams.

### EXAMPLE 4:

The potassium salt of N-(2-hydroxyethyl)-N-methylglycine of the following formula has been prepared as follows: 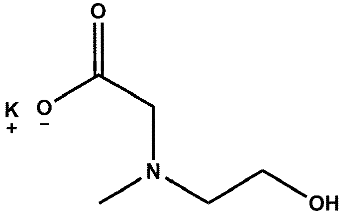
1) 72.1 g (1 mole) of 2-(methylamino)ethanol were diluted in 200 mL of distilled water and charged in a 1 L three necked flask equipped with a thermometer, condenser and a magnetic stirring bar. Then, 145.2 g (1 mole) of glyoxal 40% wt solution in water was added at room temperature through a dropping funnel. The mixture was then heated up at a temperature of about 70°C and stirred for about 16 hours (Yield 95%).
2) The second step was the neutralization of the acid obtained in the first reaction step by the addition of 106.6 g of potassium hydroxide (KOH), a 50% wt solution of potassium hydroxide (KOH) in water as the alkali hydroxide.

The resulting glycinate salt is solubilized in diethylene glycol (DEG) to obtain a solution of glycinate salt in DEG for use as a catalyst in the process of production of PIR/PUR foams.

### EXAMPLE 5: The potassium salt of dibutylglycine of the following formula has been prepared as follows:

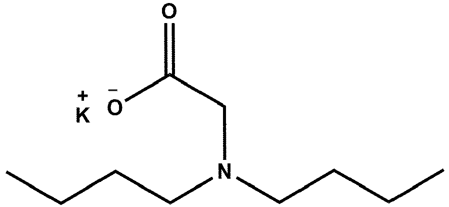

1) 129.3 g (1 mole) of dibutylamine were diluted in 200 mL of distilled water and charged in a 1 L three necked flask equipped with a thermometer, condenser and a magnetic stirring bar. Then, 145.2 g (1 mole) of glyoxal 40% wt solution in water was added at room temperature through a dropping funnel. The mixture was then heated up at a temperature of about 70°C and stirred for about 16 hours (Yield 95%).
2) The second step was the neutralization of the acid obtained in the first reaction step by the addition of 106.6 g of potassium hydroxide (KOH), a 50% wt solution of potassium hydroxide (KOH) in water as the alkali hydroxide.

The resulting glycinate salt is solubilized in diethylene glycol (DEG) to obtain a solution of glycinate salt in DEG for use as a catalyst in the process of production of PIR/PUR foams.

### EXAMPLE 6: The potassium salt of 2-(2-azabicyclo[2.2.1]heptane-2-yl)acetic acid of the following formula has been prepared as follows:

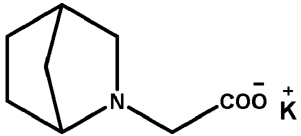

1) 75 g (1 mole) of glycine and 113.6 g formalin (37% in water, 1.4 mole formaldehyde) are charged in a 1 L three necked flask equipped with a thermometer, condenser and a magnetic stirring bar. Then, 132.2 g (2 mole) of cyclopentadiene - freshly prepared by cracking the cyclopentadiene dimer - were added at room temperature through a dropping funnel. The mixture was then vigorously stirred at room temperature for about 16 hours and afterwards the water removed in *vacuo.* The residue was then recrystallized in acetone from a concentrated ethanol solution to give 2-(2-azabicyclo[2.2.1]hept-5-en-2-yl)acetic acid as a white solid material.
2) 153.2 g (1 mole) of 2-(2-azabicyclo[2.2.1]hept-5-en-2-yl)acetic acid are dissolved in 500 mL of ethanol. Then 20 g of palladium supported on carbon (5% Pd on carbon) were added to the solution and the resulting slurry transferred in a stainless steel pressurized vessel. The material was hydrogenated at room temperature overnight at elevated hydrogen pressure.
3) The third and last step was the neutralization of the acid obtained from the previous step by the addition of 112.2 g of potassium hydroxide (KOH), a 50% wt solution of potassium hydroxide (KOH) in water as the alkali hydroxide. The potassium salt was afterwards concentrated in *vacuo.*

The resulting glycinate salt is solubilized in diethylene glycol (DEG) to obtain a solution of glycinate salt in DEG for use as a catalyst in the process of production of PIR/PUR foams.

### APPLICATION (FOAM) EXAMPLES

The rigid PIR/PUR foams were formulated as in Table 1 below wherein the components can be described as follows:

**DALTOLAC^{®} 260 polyol:** a sucrose based polyol with a hydroxyl value (mg KOH/g) of about 310, obtained from Huntsman Corporation.

**DALTOLAC^{®} 251 polyol:** a glycerine based polyol with a hydroxyl value (mg KOH/g) of about 250, obtained from Huntsman Corporation.

**VORASURF^{®} DC-193 silicone surfactant:** a silicone surfactant, obtained from Dow.

**HFC-245fa blowing agent:** 1,1,1,3,3-pentafluoropropane, a blowing agent, obtained from Honeywell International Inc.

**Catalyst LB:** a potassium acetate solution in DEG, obtained from Huntsman Corporation.

**JEFFCAT^{®} TR-90 catalyst:** 1,3,5-tris(3-(dimethylamino)propyl)-hexahydro-s-triazine, a back-end cure catalyst, obtained from Huntsman Corporation.

**JEFFCAT^{®} TR-52 catalyst:** a sodium salt of the N,N-(2-hydroxyphenyl) methylglycine chemical family.

**SUPRASEC^{®} 5025 isocyanate:** a polymeric 4,4'-methylened(phenyl isocyanate) (MDI) with an NCO content of about 31.5 wt.%, obtained from Huntsman Corporation.

The foams were prepared according to the following procedure:
The polyol blend (which includes the polyether and/or polyester polyols having a functionality between 2 and 6, water, silicone surfactant, x-linker, fire retardant and the physical blowing agent) is first pre-mixed with the catalyst for 30 seconds at low speed (500 rpm) into a carton cup of 800 ml.

Afterwards, the MDI is added and the resultant reactive solution is mixed at high speed (2000 rpm) for 5 seconds. The reacting mixture is then poured into another carton cup and the reaction profile is measured (cream time, full cup time, string/gel time, end of rise and tack free time). The detailed formulations are shown in Table 1 below.

### Table 1:

**Table 1.**

| | Comparative 1 | | Comparative 2 | | Comparative 3 | | Example 1 | | Example 3 | | Example 4 | | Example 5 | |
|---|---|---|---|---|---|---|---|---|---|---|---|---|---|---|
| polyol blend | solvent blown | water blown | solvent blown | water blown | solvent blown | water blown | solvent blown | water blown | solvent blown | water blown | solvent blown | water blown | solvent blown | water blown |
| DALTOLAC^{®} R260 polyol | 42.5 | 48.0 | 42.5 | 48.0 | 42.5 | 48.0 | 42.5 | 48.0 | 42.5 | 48.0 | 42.5 | 48.0 | 42.5 | 48.0 |
| DALTOLACO R251 polyol | 42.5 | 48.0 | 42.5 | 48.0 | 42.5 | 48.0 | 42.5 | 48.0 | 42.5 | 48.0 | 42.5 | 48.0 | 42.5 | 48.0 |
| VORASURF^{®} DC-193 Silicone surfactant | 0.5 | 0.5 | 0.5 | 0.5 | 0.5 | 0.5 | 0.5 | 0.5 | 0.5 | 0.5 | 0.5 | 0.5 | 0.5 | 0.5 |
| HFC 245 fa | 8.5 | | 8.5 | | 8.5 | | 8.5 | | 8.5 | | 8.5 | | 8.5 | |
| Water | 0.6 | 1.5 | 0.6 | 1.5 | 0.6 | 1.5 | 0.6 | 1.5 | 0.6 | 1.5 | 0.6 | 1.5 | 0.6 | 1.5 |
| SUPRASEC^{®} 5025 isocyanate | **115.0** | **150.0** | **115.0** | **150.0** | **115.0** | **150.0** | **115.0** | **150.0** | **115.0** | **150.0** | **115.0** | **150.0** | **115.0** | **150.0** |
| **Catalysts** | | | | | | | | | | | | | | |
| Catalyst LB | 0.40 | 0.45 | | | | | | | | | | | | |
| JEFFCAT^{®} TR-90 catalyst | | | 0.65 | 0.55 | | | | | | | | | | |
| JEFFCAT^{®} TR-52 catalyst | | | | | 5.00 | 5.00 | | | | | | | | |
| Example 1 | | | | | | | 5.00 | 5.00 | | | | | | |
| Example 3 | | | | | | | | | 2.50 | 2.50 | | | | |
| Example 4 | | | | | | | | | | | 5.00 | 5.00 | | |
| Example 5 | | | | | | | | | | | | | 5.00 | 5.00 |

The foams were further analyzed by Fourier Transformation Infrared Spectrometry (FTIR) to evaluate the PIR/PUR ratio. The results are summarized in Table 2 below:

**Table 2**

| **Application (Foam) Examples** | **PIR/PUR ratio water blown** | **PIR/PUR ratio solvent blown** |
|---|---|---|
| COMPARATIVE 1 | 1.05 | 1.00 |
| COMPARATIVE 2 | 0.82 | 0.70 |
| COMPARATIVE 3 | 1.29 | 1.10 |
| EXAMPLE 1 | 1.15 | 1.02 |
| EXAMPLE 3 | 1.13 | 1.05 |
| EXAMPLE 4 | 1.14 | 0.95 |
| EXAMPLE 5 | 1.08 | 1.01 |

The foam samples of the present disclosure (Examples 1 to 5) are able to provide a material with a PIR/PUR ratio comparable to the one obtainable from JEFFCAT^{®} TR-52 catalyst (Comparative Example 3) and JEFFCAT^{®} TR-90 catalyst (Comparative Example 2), both in water and solvent blown systems, demonstrating a distinct ability to act as a PIR/PUR catalyst.

## Claims

1. A process for the synthesis of a compound according to general formula (I) : 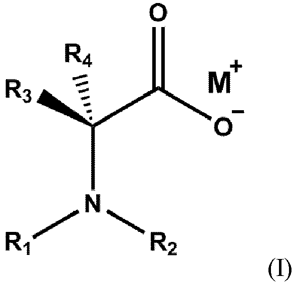 wherein
- R₁ and R₂ are independently selected from a C₁-C₁₈ straight-chain or branched alkyl group, unsubstituted or substituted with one or more hydroxyl, amino or aminoalkyl groups, or R₁ and R₂, taken together, form a 5- or 6-membered ring or 7-membered bicyclic structure, one of the members of the ring or bicyclic structure being X, wherein X is selected from CH₂, O, S, NCH₃ or NCH₂COOM,
- R₃ and R₄ are independently selected from hydrogen or a C₁-C₄ straight-chain or branched alkyl group, and
- M is an alkali metal ion or a quaternary ammonium ion
the process comprising the steps:
(i) reacting a secondary amine having the general formula R₁-NH-R₂ and a dicarbonyl compound of the general formula R₃-CO-CO-R₄ under appropriate reaction conditions to form a N,N-disubstituted glycine compound, and
(ii) neutralizing the reaction product of step (i) by adding a solution of an alkali or tetraalkylammonium hydroxide having the general formula MOH to form a compound according to general formula (I)

2. The process according to claim 1, wherein R₃ and R₄ are both hydrogen.

3. The process according to claim 1 or 2, wherein R₁ and R₂ are independently selected from an unsubstituted or substituted C₁-C₄ alkyl group.

4. The process according to any of the preceding claims, wherein R₁ and/or R₂ comprise at least one terminal hydroxyl, amino or amino-alkyl group.

5. The process according to any of the preceding claims, wherein R₁ and R₂ are identical.

6. The process according to claim 5, wherein R₁ and R₂ are both 2-hydroxyethyl.

7. The process according to claim 1, wherein a ring of the compound according to general formula (I) is selected from a 5- or 6-membered ring or 7-membered bicyclic structure with X being CH₂, a 6-membered ring with X being O or a 6-membered ring with X being NCH₂COOM.

8. The process according to any of the preceding claims, wherein M is an alkali metal ion.

9. The process according to claim 8, wherein M is a potassium ion.

10. The process according to any of the preceding claims, wherein the compound of general formula (I) is selected from potassium *N,N-*bis(2-hydroxyethyl)glycinate, potassium *N-*(2-hydroxyethyl)-*N*-methyl-glycinate, potassium *N,N*-bis(3-(dimethylamino)propyl)glycinate, potassium *N,N*-dibutyl-glycinate or combinations thereof.

11. The process according to claim 10, wherein the compound of general formula (I) is potassium *N,N*-bis(2-hydroxyethyl)glycinate for PIR/PUR foam production.

12. The process according to any of claims 1 to 9, wherein the compound of general formula (I) is selected from potassium 2-(pyrrolidin-1-yl)acetate, potassium 2-morpholinoacetate, potassium 2,2'-(piperazine-1,4-diyl)-diacetate potassium 2-(2-azabicyclo[2.2.1]heptane-2-yl)acetate or combinations thereof.

13. Use of a compound as defined in any of claims 1 to 12 as a catalyst or co-catalyst in a rigid, semi-rigid or flexible PIR/PUR foam.

14. Use according to claim 13, wherein the compound according to general formula (I) is potassium *N,N-*bis(2-hydroxyethyl)glycinate.

15. A process for producing a rigid, semi-rigid or flexible PIR/PUR foam comprising reacting a polyester polyol or polyester polyol blend with 4,4'-methylene-diphenylisocyanate (MDI) in the presence of a catalyst produced according to the process of any of claims 1 to 12 and optionally further additives under appropriate reaction conditions.

## Patentansprüche

1. Verfahren zur Synthese einer Verbindung gemäß allgemeiner Formel (I): 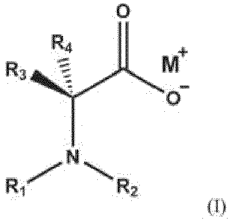 wobei
- R₁ und R₂ unabhängig ausgewählt sind aus einer geradkettigen oder verzweigten C₁-C₁₈-Alkylgruppe, unsubstituiert oder substituiert mit einer oder mehreren Hydroxyl-, Amino- oder Aminoalkylgruppen, oder R₁ und R₂, zusammengenommen, einen 5- oder 6-gliedrigen Ring oder eine 7-gliedrige bicyclische Struktur bilden, wobei eines der Glieder des Rings oder der bicyclischen Struktur X ist, wobei X ausgewählt ist aus CH₂, O, S, NCH₃ oder NCH₂COOM,
- R₃ und R₄ unabhängig ausgewählt sind aus Wasserstoff oder einer geradkettigen oder verzweigten C₁-C₄-Alkylgruppe, und
- M ein Alkalimetall-lon oder ein quaternäres Ammonium-Ion ist,
wobei das Verfahren die Schritte umfasst:
(i) Umsetzen eines sekundären Amins mit der allgemeinen Formel R₁-NH-R₂ und einer Dicarbonylverbindung der allgemeinen Formel R₃-CO-CO-R₄ unter geeigneten Reaktionsbedingungen, um eine N,N-disubstituierte Glycinverbindung zu bilden, und
(ii) Neutralisieren des Reaktionsproduktes von Schritt (i) durch Zugeben einer Lösung eines Alkali- oder Tetraalkylammoniumhydroxids mit der allgemeinen Formel MOH, um eine Verbindung gemäß allgemeiner Formel (I) zu bilden.

2. Verfahren nach Anspruch 1, wobei R₃ und R₄ beide Wasserstoff sind.

3. Verfahren nach Anspruch 1 oder 2, wobei R₁ und R₂ unabhängig ausgewählt sind aus einer unsubstituierten oder substituierten C₁-C₄-Alkylgruppe.

4. Verfahren nach einem der vorangehenden Ansprüche, wobei R₁ und/oder R₂ wenigstens eine terminale Hydroxyl-, Amino- oder Aminoalkylgruppe umfassen.

5. Verfahren nach einem der vorangehenden Ansprüche, wobei R₁ und R₂ identisch sind.

6. Verfahren nach Anspruch 5, wobei R₁ und R₂ beide 2-Hydroxyethyl sind.

7. Verfahren nach Anspruch 1, wobei ein Ring der Verbindung gemäß allgemeiner Formel (I) ausgewählt ist aus einem 5- oder 6-gliedrigen Ring oder einer 7-gliedrigen bicyclischen Struktur, wobei X CH₂ ist, einem 6-gliedrigen Ring, wobei X O ist, oder einem 6-gliedrigen Ring, wobei X NCH₂COOM ist.

8. Verfahren nach einem der vorangehenden Ansprüche, wobei M ein Alkalimetall-lon ist.

9. Verfahren nach Anspruch 8, wobei M ein Kalium-Ion ist.

10. Verfahren nach einem der vorangehenden Ansprüche, wobei die Verbindung von allgemeiner Formel (I) ausgewählt ist aus Kalium-N,N-bis(2-hydroxyethyl)glycinat, Kalium-N-(2-hydroxyethyl)-N-methyl-glycinat, Kalium-N,N-bis(3-(dimethylamino)propyl)glycinat, Kalium-N,N-dibutylglycinat oder Kombinationen davon.

11. Verfahren nach Anspruch 10, wobei die Verbindung von allgemeiner Formel (I) Kalium-N,N-bis(2-hydroxyethyl)glycinat ist, für PIR-/PUR-Schaumherstellung.

12. Verfahren nach einem der Ansprüche 1 bis 9, wobei die Verbindung von allgemeiner Formel (I) ausgewählt ist aus Kalium-2-(pyrrolidin-1-yl)acetat, Kalium-2-morpholinoacetat, Kalium-2,2'-(piperazin-1,4-diyl)diacetat, Kalium-2-(2-azobicyclo[2.2.1]heptan-2-yl)acetat oder Kombinationen davon.

13. Verwendung einer Verbindung, wie definiert in einem der Ansprüche 1 bis 12, als ein Katalysator oder Co-Katalysator in einem starren, halbstarren oder flexiblen PIR-/PUR-Schaum.

14. Verwendung nach Anspruch 13, wobei die Verbindung gemäß allgemeiner Formel (I) Kalium-N,N-bis(2-hydroxyethyl)glycinat ist.

15. Verfahren zur Herstellung eines starren, halbstarren oder flexiblen PIR-/PUR-Schaumes, umfassend das Umsetzen eines Polyesterpolyols oder Polyesterpolyol-Gemisches mit 4,4'-Methylendiphenylisocyanat (MDI) in Gegenwart eines Katalysators, hergestellt gemäß dem Verfahren nach einem der Ansprüche 1 bis 12, und fakultativ weiterer Additive unter geeigneten Reaktionsbedingungen.

## Revendications

1. Procédé pour la synthèse d'un composé selon la formule générale (I) : 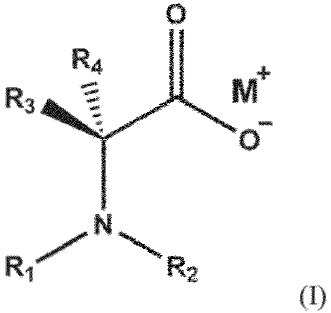 dans lequel
- R₁ et R₂ sont indépendamment sélectionnés parmi un groupe alkyle à chaîne droite ou ramifiée en C₁-C₁₈, non substitué ou substitué par un ou plusieurs groupes hydroxyle, amino ou aminoalkyle, ou R₁ et R₂, pris conjointement, forment une structure cyclique à 5 ou 6 chaînons ou bicyclique à 7 chaînons, l'un des membres de la structure cyclique ou bicyclique étant X, X étant sélectionné parmi CH₂, O, S, NCH₃ ou NCH₂COOM,
- R₃ et R₄ sont indépendamment sélectionnés parmi de l'hydrogène ou un groupe alkyle à chaîne droite ou ramifiée en C1-C₄, et
- M est un ion de métal alcalin ou un ion d'ammonium quaternaire
le procédé comprenant les étapes de :
(i) réaction d'une amine secondaire présentant la formule générale R₁-NH-R₂ et d'un composé dicarbonyle de la formule générale R₃-CO-CO-R₄ dans des conditions de réaction appropriées pour former un composé de glycine N,N-disubstitué, et
(ii) neutralisation du produit de réaction de l'étape (i) par l'ajout d'une solution d'un hydroxyde alcalin ou de tétraalkylammonium présentant la formue générale MOH pour former un composé selon la formule générale (I).

2. Procédé selon la revendication 1, dans lequel R₃ et R₄ sont tous deux de l'hydrogène.

3. Procédé selon la revendication 1 ou 2, dans lequel R₁ et R₂ sont indépendamment sélectionnés parmi un groupe alkyle en C₁-C₄ non substitué ou substitué.

4. Procédé selon l'une quelconque des revendications précédentes, dans lequel R₁ et/ou R₂ comprennent au moins un groupe hydroxyle, amino ou amino-alkyle terminal.

5. Procédé selon l'une quelconque des revendications précédentes, dans lequel R₁ et R₂ sont identiques.

6. Procédé selon la revendication 5, dans lequel R₁ et R₂ sont tous deux du 2-hydroxyéthyle.

7. Procédé selon la revendication 1, dans lequel un cycle du composé selon la formule générale (I) est sélectionné parmi une structure cyclique à 5 ou 6 chaînons ou une stucture bicyclique à 7 chaînons avec X étant du CH₂, un cycle à 6 chaînons avec X étant de l'O ou un cycle à 6 chaînons avec X étant du NCH₂COOM.

8. Procédé selon l'une quelconque des revendications précédentes, dans lequel M est un ion de métal alcalin.

9. Procédé selon la revendication 8, dans lequel M est un ion de potassium.

10. Procédé selon l'une quelconque des revendications précédentes, dans lequel le composé de formule générale (I) est sélectionné à partir de potassium *N,N*-bis(2-hydroxyéthyl)glycinate, potassium *N*-(2-hydroxyéthyl)-*N*-méthyl-glycinate, potassium *N,N*-bis(3-diméthylamino)propyl)glycinate, potassium *N,N*-dibutyl-glycinate ou des combinaisons de ceux-ci.

11. Procédé selon la revendication 10, dans lequel le composé de formule généale (I) est du potassium *N,N*-bis(2-hydroxyéthyl)glycinate pour la production de mousse PIR/PUR.

12. Procédé selon l'une quelconque des revendications 1 à 9, dans lequel le composé de formule générale (I) est sélectionné à partir de potassium 2-(pyrrolidine-1-yl)acétate, potassium 2-morpholino-acétate, potassium 2,2'-(pipérazine-1,4-diyl)-diacétate potassium 2-(2-azabicyclo[2.2.1]heptane-yl)acétate ou des combinaisons de ceux-ci.

13. Utilisation d'un composé tel que défini dans l'une quelconque des revendications 1 à 12 tel qu'un catalyseur ou co-catalyseur dans une mousse PIR/PUR rigide, semi-rigide ou flexible.

14. Utilisation selon la revendication 13, dans lequel le composé selon la formule générale (I) est du potassium *N,N*-bis(2-hydroxyéthyl)glycinate.

15. Procédé de production d'une mousse PIR/PUR rigide, semi-rigide ou flexible comprenant la réaction d'un polyol polyester ou d'un mélange de polyol polyester avec du 4,4'-diisocyanate de diphénylméthylène (MDI) en la présence d'un catalyseur produit selon le procédé selon l'une quelconque des revendications 1 à 12 et optionnellement d'autres additifs dans des conditions de réaction appropriées.
